# FASCICULE DE BREVET EUROPEEN

(11) **EP 1 246 569 B1**
(45) Date de publication et mention de la délivrance du brevet: **10.11.2004**
(21) Numéro de dépôt: 01903857.9
(22) Date de dépôt: 03.01.2001
(51) Int. Cl.: A61B 7/02, A61B 19/08

(54) **DISPOSITIF DE PROTECTION POUR INSTRUMENT MEDICAL TEL QUE STETHOSCOPE**
SCHUTZEINRICHTUNG EINES MEDIZINISCHEN INSTRUMENTES WIE Z.B. EINES STETHOSKOPS
PROTECTIVE DEVICE FOR MEDICAL INSTRUMENT SUCH AS A STETHOSCOPE

(30) Priorité: 04.01.2000 FR 0000031
(43) Date de publication de la demande: 09.10.2002
(73) Titulaire: El Massioui, Youssef, 93160 Noisy le Grand (FR); El Massioui, Farid, 91640 Briis sous Forges (FR)
(72) Inventeur: El Massioui, Youssef, 93160 Noisy le Grand (FR); El Massioui, Farid, 91640 Briis sous Forges (FR)
(74) Mandataire: Pontet, Bernard
(86) Numéro de dépôt international: PCT/FR2001/000012
(87) Numéro de publication internationale: WO 2001/049179

(56) Documents cités:
- WO-A-99/04693
- US-A- 5 090 410
- US-A- 5 564 431
- US-A- 5 587 561
- US-A- 5 747 751
- US-A- 6 006 856
- PATENT ABSTRACTS OF JAPAN vol. 1998, no. 06, 30 avril 1998 (1998-04-30) & JP 10 033528 A (SAITO FUMIKO), 10 février 1998 (1998-02-10)

## Description

La présente invention concerne un dispositif de protection pour instrument médical tel que stéthoscope.

Un tel dispositif de protection sert à protéger l'instrument médical du contact direct avec le corps humain pour éviter que l'instrument médical soit contaminé par ce contact et devienne à son tour un agent de contamination pour les patients suivants ou pour le médecin.

On connaît d'après le US-A-5 587 561 un tel dispositif de protection comprenant une paroi plane en forme de disque destinée à être appliquée sur une face circulaire d'interaction entre l'instrument et le corps du patient. Cette paroi comporte un pourtour équipé de moyens adhésifs pour l'assujettissement du dispositif de protection sur le pourtour de la face d'interaction du stéthoscope.

Le WO-A-99 04 693 décrit un dispositif analogue mais ou le moyen de fixation est constitué par un rebord périphérique permettant à la paroi d'être mise en place à la manière d'un capuchon.

Ces dispositifs connus ont l'inconvénient d'être très liés à une dimension particulière de la face active du stéthoscope. En particulier, le WO-A-99 04 693 prévoit deux dispositifs différents pour les deux faces opposées, de diamètre différent, habituellement prévues sur un stéthoscope. En outre, la mise en place des dispositifs de protection connus sur la face d'interaction du stéthoscope nécessite une manipulation minutieuse d'une étroite région périphérique entourant la paroi. Les moyens de fixation par adhésif sont mal commodes et finissent par laisser des traces d'adhésif sur le stéthoscope. Ces traces d'adhésifs collectent à la longue des contaminants inertes ou microbiens.

Le US-A-5 564 431 décrit un dispositif de protection avec une paroi en forme de sac qui enveloppe toute la zone d'interaction de l'instrument avec le corps du patient. Les moyens d'attache sont disposés autour d'une ouverture du sac pour serrer élastiquement cette ouverture, autour du tube de l'instrument. La protection ainsi proposée est intégrale. Le praticien n'a donc pas à protéger séparément les deux faces d'interaction de l'instrument ni à prévoir à l'avance quelle face d'interaction sera utilisée avec chaque patient, ni à prévoir deux stocks différents pour les deux faces. Ce type de dispositif de protection est plus facile à mettre en place d'un simple geste, plus stable en position, et plus efficace car recouvrant plus largement la zone d'interaction de l'instrument avec le corps du patient.

Le but de la présente invention est ainsi de proposer une protection pour instrument médical tel que stéthoscope qui soit quasiment universelle, plus efficace et plus facile à installer que les dispositifs connus.

Suivant l'invention, le dispositif de protection pour instrument médical tel que stéthoscope, pour protéger une zone d'interaction de l'instrument avec le corps du patient, comprenant des moyens à paroi souple conçus pour recouvrir simultanément deux faces d'interaction avec le corps, et des moyens d'attache pour assujettir ladite paroi contre ladite face, est caractérisé en ce que les moyens à paroi souple sont agencés pour être disposés en U sur la zone d'interaction de l'instrument en couvrant simultanément deux faces d'interaction opposées de l'instrument.

Suivant l'invention, les moyens à paroi souple sont agencés pour être disposés en U sur la zone d'interaction de l'instrument de façon à couvrir simultanément deux faces d'interaction opposées de l'instrument. La mise en place peut être simple et fiable, d'abord par accrochage d'une extrémité de la paroi souple puis, après formation du U, par accrochage de l'autre extrémité de la paroi souple, le tout quasiment d'un seul geste réalisable d'une seule main.

D'autres particularités et avantages de l'invention ressortiront encore de la description ci-après, relative à des exemples non-limitatifs.

Aux dessins annexés :
- la figure 1 est une vue en perspective d'un stéthoscope, équipée d'un dispositif de protection ne faisant pas partie de l'invention;
- la figure 2 est une vue en élévation de la tête du stéthoscope, avec coupe du dispositif de protection;
- la figure 3 est une vue en élévation de la tête du stéthoscope équipé d'un premier mode de réalisation du dispositif selon l'invention;
- la figure 4 est une vue en plan du dispositif de protection de la figure 3 avant mise en place; et
- les figures 5 et 6 sont des vues analogues aux figures 3 et 4 respectivement, mais relatives à un deuxième mode de réalisation de l'invention.

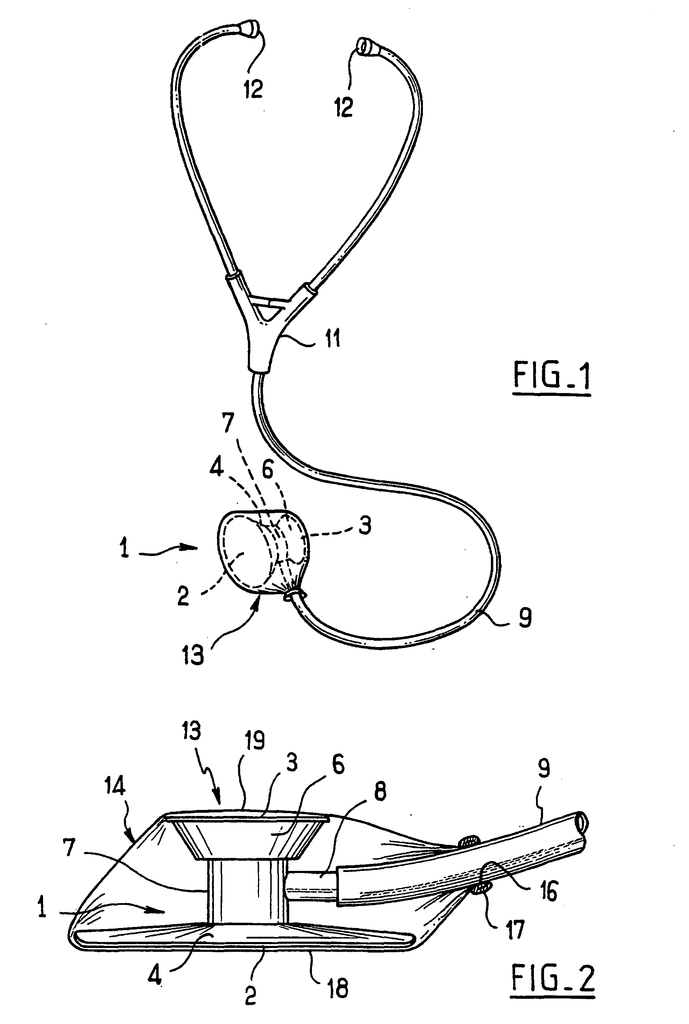

De manière classique, le stéthoscope représenté à la figure 1 comprend une zone 1 d'interaction avec le corps du patient, constituée par une tête d'auscultation comprenant deux faces d'interaction opposées 2 et 3. La face d'interaction 2, de relativement grand diamètre sert à capter les fréquences sonores relativement grandes. La face d'interaction 3, constituée par une sorte de cloche de diamètre extérieur plus petit que la face 2, est destinée à capter les fréquences plus basses, de l'ordre de quelques dizaines d'hertz. Selon les phénomènes ou symptômes recherchés, le praticien applique l'une ou l'autre des faces 2 et 3 contre le corps du patient, en particulier, contre sa poitrine. Les deux faces 2 et 3 sont supportées par des coupelles tronconiques respectives 4 et 6 qui à partir des faces d'interaction 2 et 3 se rétrécissent l'une vers l'autre pour rejoindre un corps central cylindrique 7 de diamètre plus petit que celui de chacune des faces 2 et 3. Le corps central 7 porte un raccord 8 (figure 2) dirigé radialement vers l'extérieur, sur lequel est emmanché un tube souple 9. Un raccord en Y 11 relie le tube 9 avec deux embouts d'écoute 12 pour les oreilles du praticien.

Le stéthoscope et plus particulièrement sa tête d'auscultation 1 sont équipés d'un dispositif de protection 13 destiné à empêcher tout contact direct entre le stéthoscope et le corps du patient, en particulier pendant l'auscultation de ce dernier avec l'une ou l'autre des faces d'interaction 2 et 3.

Dans la réalisation représentée aux figures 1 et 2 qui ne fait pas partie de l'invention, le dispositif de protection 13 comprend une paroi souple en forme de sac 14 qui emmaillote complètement la zone d'interaction 1, de manière sensiblement étanche et continue, à la manière d'un capuchon. La paroi en forme de sac 14 comprend une ouverture 16 entourée par un bourrelet élastique 17 formant le bord de la paroi en forme de sac 14. Le bourrelet élastique 17 tend en permanence à quasiment refermer l'ouverture 16, mais présente une extensibilité suffisante pour permettre d'introduire la tête d'auscultation 1 à travers l'ouverture 16. Après cette introduction, le bourrelet 17 enserre élastiquement le conduit 9 en assurant à la fois le maintien en position de la paroi en forme de sac 14 autour de la tête d'auscultation 1 et une fermeture quasiment étanche pour l'espace situé à l'intérieur de la paroi en forme de sac 14.

Lorsque le dispositif de protection est installé sur la tête d'auscultation comme représenté aux figures 1 et 2, la paroi souple 14 en forme de sac présente deux régions 18, 19 qui recouvrent la totalité des faces d'interaction 2 et 3 respectivement, en prenant une configuration sensiblement plane.

A la figure 2, c'est pour clarifier le dessin qu'on a représenté un espace entre les régions 18 et 19 de la paroi 14 et les faces correspondantes 2 et 3 de la tête d'auscultation 1. En pratique, les régions 18 et 19 sont appliquées en appui contre les faces 2 et 3, ou tout au moins contre leur pourtour.

Pour permettre ce résultat, on préfère que la paroi souple 14 soit extensible et soit soumise à une assez forte extension élastique lorsqu'elle est en place sur la tête d'auscultation 1 comme le représentent les figures 1 et 2. Ainsi, la paroi 14 ne constitue pas une gêne pendant l'auscultation, ni pour le confort du patient ni pour la fidélité de la transmission sonore jusqu'aux oreilles du médecin.

De préférence, la paroi 14 est réalisée en latex avec une épaisseur aussi réduite que possible. D'autres matières possibles sont du papier, du film plastique extensible ou non. La bonne transmission sonore constitue un critère de choix du matériau.

Une fois l'auscultation d'un patient terminée, le praticien ôte le dispositif de protection 14 et le jette. Il utilise un dispositif de protection neuf pour le patient suivant.

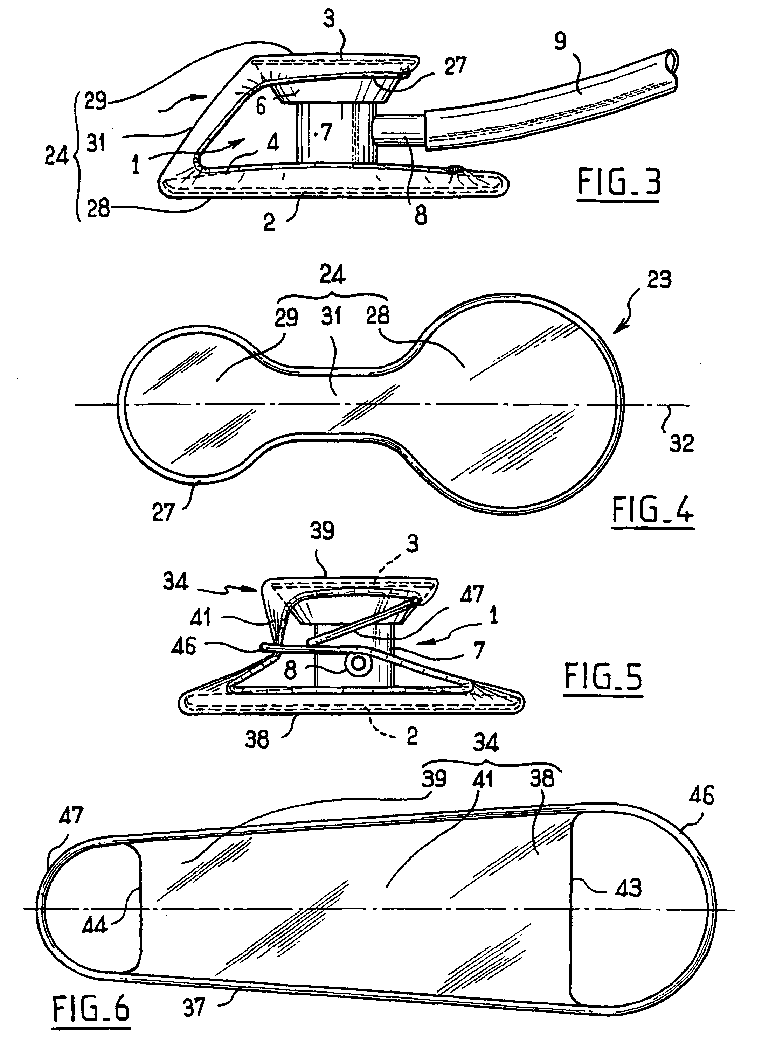

Dans le premier mode de réalisation de l'invention, représenté aux figures 3 et 4, et qui ne sera décrit que pour ses différences par rapport à la réalisation des figures 1 et 2, le dispositif de protection 23 a une forme générale allongée semblable à celle d'un diabolo dissymétrique (figure 4). La paroi souple 24 comprend une grande région essentiellement circulaire 28 pour recouvrir la grande face d'interaction 2 de la tête 1, une petite région essentiellement circulaire 29 pour recouvrir la petite face d'interaction 3 de la tête 1, et une bande de raccordement 31 qui relie les deux régions 28 et 29 entre elles en s'étendant sensiblement le long d'une ligne diamétrale 32 commune à ces deux régions. La paroi souple en forme de diabolo est entourée sur tout son pourtour par un bourrelet élastique 27.

En service, comme illustré à la figure 3, on place les régions circulaires 28 et 29 de la paroi 24 sur les faces d'interaction 2 et 3 respectivement de la tête 1, le dispositif de protection 23, tel que vu en élévation comme à la figure 3, étant disposé en U avec la région 31 constituant la barre de liaison inférieure du U.

Par un choix judicieux du périmètre défini par le bourrelet 27 lorsqu'il est au repos, on obtient en service la situation représentée à la figure 3 où le bourrelet 27 forme un rebord venant en prise autour d'une partie de la périphérie des coupelles tronconiques 4 et 6 formant un retrait derrière les faces 2 et 3. La contrainte de tension élastique du bourrelet fait que les deux régions circulaires 28 et 29 sont tendues en configuration plane sur les faces d'interaction 2 et 3 respectives.

La mise en place de ce dispositif de protection s'effectue très simplement en accrochant le bourrelet 27 par l'une des extrémités longitudinales du dispositif de protection contre la paroi tronconique 4 ou 6 correspondante, par exemple dans la zone située au-dessus du raccord 8, puis en étirant le dispositif de protection 23 et en le recourbant en U jusqu'à pouvoir accrocher son autre extrémité longitudinale sur l'autre coupelle tronconique 4 ou 6 de l'autre côté du raccord 8. On relâche ensuite le bourrelet 27 qui en se rétractant élastiquement assure le positionnement stable du dispositif sur la tête 1.

L'exemple des figures 5 et 6 constituant un deuxième mode de réalisation de l'invention ne sera décrit que pour ses différences par rapport à celui des figures 3 et 4.

La paroi souple 34 (figure 6) a maintenant la forme d'un trapèze allongé regroupant la région 38 destinée à recouvrir la face d'interaction 2 et adjacente à la grande base 43 du trapèze, la région 39 destinée à recouvrir la petite face d'interaction 3 de la tête 1 et adjacente à la petite base 44 du trapèze, et la région de liaison 41 qui relie les régions 38 et 39.

Contrairement à la réalisation des figures 3 et 4, la région de liaison 41 des figures 5 et 6 ne forme plus un rétrécissement entre les régions 38 et 39.

Une autre différence est que le dispositif a une plus grande longueur totale. Le bourrelet élastique 37 s'étend le long des deux côtés longitudinaux de la paroi souple trapézoïdale 34 mais s'écarte des deux bases 43 et 44 pour former au-delà de chacune d'elles un arceau d'accrochage respectif 46, 47. Comme le montre la figure 5, on met en place le dispositif de protection en engageant le petit arceau 47 autour du corps central 7 de la tête d'auscultation 1 après avoir fait passer la petite face d'interaction 3 à travers l'arceau 47, puis on place la région 39 sur la face 3, on recourbe en U le dispositif de protection pour placer la région 38 sur la grande face d'interaction 2, puis on replie encore une fois le dispositif pour faire passer à travers le grand arceau 46 la face de protection 3 maintenant recouverte par la région 39 de la paroi souple 34. Comme le montre encore la figure 5, le résultat obtenu est que la région de liaison 41 se trouve à l'intérieur de l'arceau 46, lequel tend élastiquement la région de liaison 41 de la paroi souple 34, ce qui améliore encore la tension mécanique souhaitable dans la paroi souple 34.

Bien-entendu, l'invention n'est pas limitée aux deux modes de réalisation décrits et représentés.

Dans l'exemple des figures 5 et 6, la paroi souple pourrait avoir sensiblement la forme de diabolo de la figure 4. A l'inverse, dans les exemples des figures 3 et 4, la région de liaison 31 pourrait ne former aucun rétrécissement par rapport aux régions de recouvrement 28 et 29.

On pourrait encore envisager un mode de réalisation dont la partie gauche correspondrait à la figure 6 avec un arceau 47 pour réaliser le premier accrochage sur le corps central 7 de la tête d'auscultation 1, et la partie droite ressemblerait à celle de la figure 4 pour simplement permettre d'accrocher le bourrelet élastique contre la coupelle tronconique 4 située derrière la grande face d'interaction 2. On pourrait encore inverser, c'est à dire réaliser un dispositif dont la partie droite ressemblerait à celle de la figure 6 et la partie gauche à celle de la figure 4.

Pour la fixation du dispositif de protection sur la tête d'auscultation 1, on pourrait remplacer le bourrelet élastique par un ourlet dans lequel circule un cordon de serrage manuel, ou encore un fil métallique.

Le bourrelet élastique 27 ou 37 des figures 3 à 6 pourrait être interrompu dans certaines zones du pourtour, par exemple le long de la région de liaison 31 ou 41.

## Revendications

1. Dispositif de protection pour instrument médical tel que stéthoscope, pour protéger une zone (1) d'interaction de l'instrument avec le corps du patient, comprenant des moyens à paroi souple (14, 24, 34) conçus pour recouvrir simultanément deux faces d'interaction (2,3) de l'instrument avec le corps, et des moyens d'attache (17, 27, 37) pour assujettir ladite paroi contre ladite face, **caractérisé en ce que** les moyens à paroi souple (24, 34) sont agencés pour être disposés en U sur la zone d'interaction (1) de l'instrument en couvrant simultanément deux faces d'interaction opposées (2, 3) de l'instrument.

2. Dispositif selon la revendication 1, **caractérisé en ce que** les moyens à paroi souple comprennent une paroi souple unique (28, 29, 31; 38, 39, 41) allongée, s'étendant en service tout le long du U.

3. Dispositif selon la revendication 1 ou 2, **caractérisé en ce que** les moyens d'attache comprennent à une des extrémités des moyens à paroi souple (34) un arceau (47) destiné à être placé autour d'un corps rétréci (7) de l'instrument, situé entre les deux faces d'interaction (2, 3) opposées.

4. Dispositif selon la revendication 3, **caractérisé en ce que** les moyens d'attache comprennent une seconde bride (46) à l'autre extrémité des moyens à paroi souple (34), de façon que la bride (46) mise en place en second lieu entoure simultanément le corps (7) de l'instrument et la partie centrale (41) en U.

5. Dispositif selon la revendication 1 ou 2, **caractérisé en ce que** les moyens d'attache (17, 27, 37) comprennent des moyens élastiquement extensibles placés le long d'une partie au moins du pourtour des moyens à paroi souple (14, 24, 34).

6. Dispositif selon l'une des revendications 1 à 4, **caractérisé en ce que** les moyens d'attache comprennent des moyens élastiques (17, 27, 37) permettant la mise en place par extension élastique et le maintien des moyens à paroi souple (14, 24, 34) en laissant les moyens élastiques se rétracter autour de faces en retrait (4, 6) de l'instrument.

7. Dispositif selon l'une des revendications 1 à 6, **caractérisé en ce que** la paroi souple (24) a une forme de diabolo dissymétrique avec une bande de raccordement (31) qui réunit deux régions (28,29) spécifiques essentiellement circulaires pour recouvrir chacune une face d'interaction.

8. Dispositif selon l'une des revendications 1 à 6, **caractérisé en ce que** la paroi souple (24) a une forme de trapèze allongé.

## Patentansprüche

1. Schutzvorrichtung für ein medizinisches Instrument wie ein Stethoskop zum Schützen einer zur Interaktion mit dem Körper des Patienten dienenden Zone (1) des Instruments, umfassend Mittel (14, 24, 34) mit einer biegsamen Wand, die ausgelegt sind, um gleichzeitig zwei zur Interaktion mit dem Körper dienende Seiten (2, 3) des Instruments zu bedekken, und Befestigungsmittel (17, 27, 37) zur Befestigung dieser Wand an dieser Seite, **dadurch gekennzeichnet, daß** die Mittel (24, 34) mit biegsamer Wand ausgebildet sind, um in einem U auf der Interaktionszone (1) des Instruments angeordnet zu werden, indem sie zwei entgegengesetzte Interaktionsseiten (2, 3) des Instruments gleichzeitig bedecken.

2. Vorrichtung nach Anspruch 1, **dadurch gekennzeichnet, daß** die Mittel mit biegsamer Wand eine einzige langgestreckte biegsame Wand (28, 29, 31; 38, 39, 41) umfassen, die sich in Betrieb längs des U's erstreckt.

3. Vorrichtung nach Anspruch 1 oder 2, **dadurch gekennzeichnet, daß** die Befestigungsmittel an einem der Enden der Mittel (34) mit biegsamer Wand einen Bügel (47) aufweisen, der dazu bestimmt ist, um einen verjüngten Körper (7) des Instruments herum angeordnet zu werden, der zwischen den beiden entgegengesetzten Interaktionsseiten (2, 3) gelegen ist.

4. Vorrichtung nach Anspruch 3, **dadurch gekennzeichnet, daß** die Befestigungsmittel am anderen Ende der Mittel (34) mit biegsamer Wand eine zweite Klammer (46) aufweisen, so daß die an zweiter Stelle aufgesetzte Klammer (46) gleichzeitig den Körper (7) des Instruments und den zentralen U-Teil (41) umgibt.

5. Vorrichtung nach Anspruch 1 oder 2, **dadurch gekennzeichnet, daß** die Befestigungsmittel (17, 27, 37) elastisch dehnbare Mittel umfassen, die längs mindestens eines Teils des Umfangs der Mittel (14, 24, 34) mit biegsamer Wand angeordnet sind.

6. Vorrichtung nach einem der Ansprüche 1 bis 4, **dadurch gekennzeichnet, daß** die Befestigungsmittel elastische Mittel (17, 27, 37) umfassen, die die Anbringung der Mittel (14, 24, 34) mit biegsamer Wand durch elastische Dehnung und ihren Halt gestatten, indem man die elastischen Mittel sich um zurückversetzte Seiten (4, 6) des Instruments herum zusammenziehen läßt.

7. Vorrichtung nach einem der Ansprüche 1 bis 6, **dadurch gekennzeichnet, daß** die biegsame Wand (24) die Form eines unsymmetrischen Diabolos mit einem Verbindungsband (31) hat, das zwei im wesentlichen kreisförmige Bereiche (28, 29) zum Bedecken jeweils einer Interaktionsseite verbindet.

8. Vorrichtung nach einem der Ansprüche 1 bis 6, **dadurch gekennzeichnet, daß** die biegsame Wand (24) die Form eines langgestreckten Trapezes hat.

## Claims

1. A protective device for a medical instrument such as a stethoscope, for protecting a zone (1) of interaction of the instrument with the body of a patient, comprising flexible wall means (14, 24, 34) adapted simultaneously to cover two interaction faces (2, 3) of the instrument with the body, and fastening means (17, 27, 37) for attaching said wall against said face, **characterized in that** the flexible wall means (24, 34) are arranged to be placed with a U-shape above the interaction zone (1) of the instrument while simultaneously covering two opposed interaction faces (2, 3) of the instrument.

2. A device according to claim 1, **characterized in that** the flexible wall means comprise an elongated single flexible wall (28, 29, 31; 38, 39, 41) which, in operation, extends along the entirety of the U.

3. A device according to claim 1 or 2, **characterized in that** the fastening means comprise a loop (47) at one of the ends of the flexible wall means (34), said loop (47) being intended to be placed about a narrowed body (7) of the instrument, said body located between both opposed interaction faces (2, 3).

4. A device according to claim 3, **characterized in that** the fastening means comprise a second loop (46) at the other end of the flexible wall means (34), so that the loop (46) which is positioned in the second place simultaneously surrounds the body (7) of the instrument and the central part (41) of the U.

5. A device according to claim 1 or 2, **characterized in that** the fastening means (17, 27, 37) comprise resiliently extensible means arranged along at least part of the periphery of the flexible wall means (14, 24, 34).

6. A device according to one of claims 1-4, **characterized in that** the fastening means comprise resilient means (17, 27, 37) allowing the flexible wall means (14, 24, 34) to be positioned by a resilient extension and to be maintained by allowing retraction of the resilient means about set back faces (4, 6) of the instrument.

7. A device according to one of claims 1-6, **characterized in that** the flexible wall (24) has a dissymmetrical diabolo shape with a connecting strip (31) which joins two regions (28, 29) which are substantially circular, each region for covering a respective interaction face.

8. A device according to one of claims 1-6, **characterized in that** the flexible wall (24) has an elongated trapezium shape.
